# EUROPÄISCHE PATENTSCHRIFT

(11) **EP 0 690 066 B1**
(45) Veröffentlichungstag und Bekanntmachung des Hinweises auf die Patenterteilung: **13.03.2002**
(21) Anmeldenummer: 95109926.6
(22) Anmeldetag: 26.06.1995
(51) Int. Cl.: C07F 9/6584, A61K 31/675

(54) **Phosphinsäure-Derivate, deren Herstellung sowie deren Verwendung**
Phosphinic acid derivatives, their preparation as well as their use
Dérivés d'acides phosphiniques, leur préparation ainsi que leur utilisation

(30) Priorität: 30.06.1994 DE 4422911
(43) Veröffentlichungstag der Anmeldung: 03.01.1996
(73) Patentinhaber: Aventis Pharma Deutschland GmbH, 65929 Frankfurt am Main (DE)
(72) Erfinder: Peyman, Anuschirwan, Dr., D-65779 Kelkheim (DE); Stahl, Wilhelm, Dr., D-65929 Frankfurt (DE); Budt, Karl-Heinz, Dr., D-65191 Wiesbaden (DE); Ruppert, Dieter, Dr., D-65812 Bad Soden (DE); Schüssler, Henning, D-65931 Frankfurt (DE); Wagner, Konrad, D-65931 Frankfurt (DE)

(56) Entgegenhaltungen:
- WO-A-92/00954
- WO-A-93/07128
- WO-A-95/02582
- BIOORG. MED. CHEM. LETT. (BMCLE8,0960894X);94; VOL.4 (21); PP.2601-4, HOECHST AG;FRANKFURT; 65926; GERMANY (DE) Peyman A et al 'Non-peptide-based inhibitors of human immunodeficiency virus-1 protease'
- JADHAV ET AL.: 'Nonpeptide cyclic cyanoguanidines as HIV-1 protease inhinbitors' J. MED. CHEM. Bd. 41, 1998, Seiten 1446 - 1455

## Beschreibung

Die vorliegende Erfindung betrifft Phosphinsäurederivate, deren Herstellung sowie deren Verwendung. Die erfindungsgemäßen Verbindungen dienen z.B. als Inhibitoren von Aspartylproteasen, insbesondere als Inhibitoren von retroviralen Proteasen, insbesondere der HIV-Protease. Pharmazeutische Kompositionen dieser Verbindungen finden Verwendung zur Behandlung solcher Krankheiten, bei denen sich durch die Inhibierung von Aspartylproteasen eine Verbesserung erzielen läßt, besonders virale Infektionen, ganz besonders Infektionen mit Retroviren, die auf eine solche Behandlung ansprechen, insbesondere bei Infektionen durch das Human Immunodeficiency Virus (HIV).

Die ethiologische Ursache des "erworbenen Immunschwäche-Syndroms" (engl: acquired immunodeficiency syndrom AIDS) ist der sogenannte Human Immunodeficiency Virus (HIV). Die Aids Epidemie hat sich mittlerweile über nahezu alle Staaten ausgebreitet. Gegenwärtige Behandlungsmethoden für AIDS beinhalten Verbindungen wie 3'-Azido-3'-deoxythymidin (AZT) 2',3'-Dideoxyinosin (ddl), 2',3'-Dideoxycytidin(ddC) und andere insbesondere nichtnukleosidische Reverse Transkriptase (RT) Inhibitoren, welche die virale DNA Synthese inhibieren. Das therapeutisch am längsten erprobte AZT bewirkt eine mittlere Verlängerung der Lebenszeit bei Vollbild von AIDS von ca. 6 auf im Mittel 24 Monate, besitzt jedoch ernste und toxische Nebeneffekte, die in vielen Fällen das Absetzen der Therapie verlangen. Ein weiteres Problem ist die rasche Entwicklung resistenter HIV Stämme, sowohl gegen AZT, wie auch gegen ddl und ddC und nichtnucleosidische RT-Inhibitoren. Weitere Ansatzpunkte in der HIV Therapie sind daher dringend erforderlich. Eines der prominentesten Targets der HIV Chemotherapie ist die sogenannte HIV Protease, eine Aspartylprotease, welche virale Polyproteine spezifisch spaltet und für den viralen Lebenszyklus essentiell ist. Die Inhibierung der Protease inhibiert auch die Virus-Replikation.

Eine Übersicht über den Stand der Technik auf dem Gebiet der HIV-Protease-Inhibitoren geben beispielsweise: J. A. Martin, Antiviral Research 17 (1992) 265; S. S. Abdel-Meguid, Med. Res. Rev. 13 (1993) 731; M. Lang, J. Roesel, Arch. Pharm. 326 (1993) 921. Die bekannten Inhibitoren der HIV-Protease lassen sich in zwei strukturelle Klassen einteilen: "Peptide-based" und "Non-Peptide-based" inhibitors.

Die sich abzeichnende Resistenzentwicklung von HIV-Stämmen auch beim Einsatz verschiedener HIV-Protease Inhibitoren und die oftmals unzureichende Bioverfügbarkeit und Pharmakokinetik, die insbesondere bei den "Peptidebased"-Inhibitoren beobachtet wird, unterstreichen den Bedarf nach neuen, vorzugsweise "Non-Peptide-based"-Strukturen (beispielsweise WO 93/07128 oder P. Y. S. Lam et al., Science 263 (1994) 380), die in der Lage sind, die HIV-Protease effektiv zu inhibieren.

In der WO-A-9200954 werden Phosphinsäure-Derivate beschrieben, die als Aspartyl-Protease-Inhibitoren wirken können.

Überraschenderweise wurde nun gefunden, daß bestimmte Phosphinsäure-Derivate hervorragende Hemmstoffe der Aspartylproteasen sind.

Gegenstand der Erfindung sind daher Verbindungen der Formel I 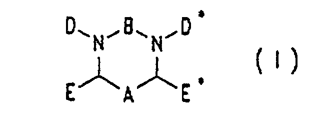 worin A für einen Rest der Formel IIa, IIb, IIc oder IId 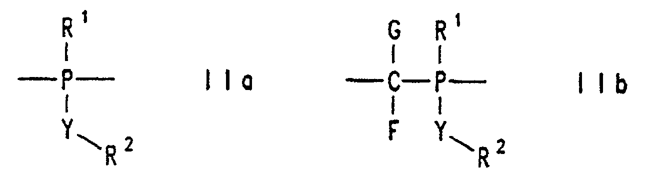 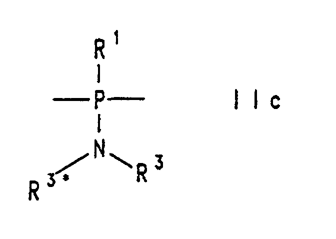 steht;
- Y: für Sauerstoff oder Schwefel steht;
- R¹: für Sauerstoff oder Schwefel steht,,
- R²: für
- Wasserstoff,
- (C₁-C₄)-Alkyl,
- ein Äquivalent eines physiologisch verträglichen Salzes, oder
- ein Rest einer Phosphinsäure-Prodrug,
steht;
- R³ und R^{3*}: Wasserstoff bedeuten;
- D und D*: unabhängig voneinander für R⁶ oder R⁶-CH₂- stehen, wobei R⁶ für (C₆-C₁₂)-Aryl, unsubstituiert oder einfach substituiert mit R⁷
wobei R⁷ unabhängig voneinander für
- R⁸
- OR⁸,
- F, -Cl, -Br steht, wobei
- R⁸ für: Wasserstoff,
(C₁-C₆)-Alkyl, unsubstituiert oder mit Hydroxy oder Br substituiert und
- E und E*: Wasserstoff oder
Benzyl bedeuten.

Eine besondere Bedeutung besitzen weiterhin die obengenannten Verbindungen der Formel I, in denen
- R²: für
- Wasserstoff;
- (C₁-C₄)-Alkyl oder
- ein Äquivalent eines physiologisch verträglichen Salzes steht,
und D steht für Phenyl oder Benzyl, substituiert mit R⁸ wobei
- R⁸: Wasserstoff,
(C₁-C₃)-Alkyl, gegebenenfalls mit Hydroxy oder Br substituiert, bedeutet.

Die nachfolgenden Beispiele für Substituenten - soweit nicht anders definiert - gelten für Substituenten in einfacher und zusammengesetzter bzw. funktionalisierter Form (z.B. Alkyl, Aryl, Alkoxy, Alkylamino etc.).

Beispiele für Alkyl sind Methyl, Ethyl, Propyl, Butyl, Pentyl, Hexyl, Heptyl, Octyl, iso-Propyl, iso-Butyl, Decyl, Dodecyl, tert-Butyl, iso-Pentyl, Allyl, Butenyl, Pentenyl, Hexenyl.

Beispiele für Aryl sind Phenyl, 1-Naphthyl, 2-Naphthyl,
Beispiele für Arylalkyl sind Benzyl, 2-Naphthylmethyl, 1-Naphthylmethyl, Phenylethyl, Phenylpropyl, 2-Naphthylethyl, 2-Naphthylpropyl, 1-Naphthylethyl, 1-Naphthylpropyl,
Beispiele für Cycloalkyl sind cyclo-Propyl, cyclo-Butyl, cyclo-pentyl, cyclo-Hexyl, cyclo-Heptyl.

Unter physiologisch verträglichen Salzen von Verbindungen der Formel I versteht man sowohl anorganische als auch organische Salze, z.B. wie sie in Remington's Pharmaceutical Sciences (17. Auflage, Seite 1418 (1985) beschrieben sind.
Aufgrund der physikalischen und chemischen Stabilität und der Löslichkeit sind für saure Gruppen unter anderem Natrium-, Kalium-, Calcium und Ammoniumsalze bevorzugt.

Unter Phosphinsäure-Prodrug sind solche Gruppen zu verstehen, die sich unter physiologischen Bedingungen in die entsprechenden Phosphinsäuren umwandeln ("Bioreversible Schutzgruppen"). Dies sind dieselben Gruppen, die dem Fachmann als Phosphat-Prodrugs bekannt sind, z.B. Acyloxyalkyl-Ester (D. Srivasta, D. Farquhar, Bioorg. Chem. 12 (1984) 118) wie beispielsweise Pivaloyloxymethyl-, Pivaloyloxyethyl-, Pivaloyloxyisobutyl, Acetyloxymethyl oder Acetyloxyethyl-Ester oder S-Acylthioethanol-Ester (C. Perigaud et al., Bioorg. Med. Chem. Lett. 3 (1993) 2521) wie beispielsweise S-Acetylthioethanolester. Unter Hydroxy-Prodrugs sind solche Gruppen zu verstehen, die sich unter physiologischen Bedingungen in die entsprechende Hydroxygruppe umwandeln ("Bioreversible Schutzgruppen"). Solche Gruppen sind dem Fachmann bekannt und beispielsweise in H. Bundgaard, Design of Prodrugs, Elsevier Science Publishers B.V., Amsterdam 1985 beschrieben.

Ein weiterer Gegenstand der Erfindung ist ein Verfahren zur Herstellung von Verbindungen der Formel I, das dadurch gekennzeichnet ist, daß (Schritt 1) eine Verbindung der Formel IV 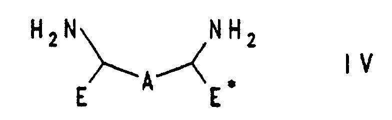 worin A, E, E* die obengenannten Bedeutungen haben, mit aktivierten Kohlensäure- oder Oxalsäurederivaten
zu zyklischen Harnstoffen bzw. zyklischen Oxalsäurediamiden der Formel V 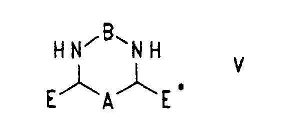 worin A, E, E* und B die oben genannte Bedeutung haben, umgesetzt wird und anschließend (Schritt 2) die erhaltenen Verbindungen zu Verbindungen der Formel I alkyliert werden oder daß die Verbindungen der Formel IV alkyliert werden und anschließend die erhaltenen Verbindungen mit aktivierten Kohlensäure oder Oxalsäurederivaten zu Verbindungen der Formel I zyklisiert werden.
Die Umsetzung erfolgt gemäß dem Fachmann bekannten Methoden (z.B. J. March, "Advanced Organic Chemistry", 3rd Ed., Wiley-Interscience, New York 1985).

Die Synthese der Verbindungen der Formel IV ist beschrieben (z.B.: A. Peyman et al., Angew. Chem. 105 (1993) 1852; B. Stowasser et al., Tetrahedron Lett. 33 (1992) 6625). In Schritt 1 werden zur Synthese der zyklischen Harnstoffe die Reagentien der Formel X-(C=L)-X' eingesetzt, wobei X und X' Abgangsgruppen sind und L wie oben definiert ist, die Reaktion wird unter verstärkter Verdünnung durchgeführt, um den Ringschluß zu ermöglichen. Beispiele für X(C=L)X' sind Carbonyldiimidazol, Thiocarbonyldiimidazol, Phosgen, Thiophosgen, Diphenylcarbonat, Diphenylthiocarbonat und Bistrichlormethylcarbonat. Zur Synthese zyklischer Oxalsäurediamide werden aktivierte Derivate der Oxalsäure eingesetzt, bevorzugt Oxalylchlorid unter den obengenannten Bedingungen.
Gegebenenfalls werden funktionelle Gruppen (nicht jedoch die endständigen Aminogruppen) in den Diaminen IV nach dem Fachmann bekannten Verfahren geschützt (z.B. Greene, Wuts, "Protective Groups in Organic Synthesis", J. Wiley, New York 1991),Hydroxygruppen beispielsweise mit Acylgruppen wie z.B. Acetyl oder Phosphinsäuren gegebenenfalls als Ester wie z.B. Methylester.

In Schritt 2 werden die Verbindungen der Formel V durch Alkylierung zu Verbindungen der Formel I umgesetzt. Auch die Alkylierung erfolgt z.B. nach dem Fachmann bekannten Verfahren durch Umsetzung mit einer Base und einem alkylierenden Reagens, gegebenenfalls unter Phasentransferkatalyse (E. V. Dehmlow, S. S. Dehmlow, "Phase Transfer Catalysis", 3rd Ed., VCH, Weinheim 1993) in einem geeigneten organischen Lösungsmittel. Bevorzugte Alkylierungsreagentien sind Verbindungen D-Y bzw. D*-Y, worin Y für eine Abgangsgruppe wie beispielsweise Halogen, Triflat oder Mesylat steht. Bevorzugte Lösungsmittel sind polar aprotische Lösungsmittel, wie beispielsweise DMSO. Gegebenenfalls werden funktionelle Gruppen in D-Y bzw. D*-Y nach dem Fachmann bekannten Verfahren geschützt (z.B. Greene, Wuts, "Protective Groups in Organic Synthesis", J. Wiley, New York 1991). Bevorzugte Schutzgruppen für die Hydroxy-Gruppen sind beispielsweise Trimethylsilylethoxymethyl (SEM), Methoxyethoxymethyl (MEM), Methoxymethyl (MOM), 4-Methoxyphenyl (MOP).
Am Ende der Synthese werden die Schutzgruppen ebenfalls nach bekannten Verfahren wieder abgespalten.

Die Synthese der Phosphinsäureamide erfolgt aus den Phosphinsäuren nach bekannten Verfahren, beispielsweise durch Überführung in die Phosphinsäurechloride und anschließende Aminolyse gegebenenfalls vor der Abspaltung der Schutzgruppen.

Weiterer Gegenstand der Erfindung ist die Verwendung der Verbindungen der Formel I als Heilmittel und pharmazeutische Präparate, die diese Verbindungen enthalten.
Pharmazeutische Präparate enthalten eine wirksame Menge des Wirkstoffs der Formel I zusammen mit einem anorganischen oder organischen pharmazeutisch verwendbaren Trägerstoff.
Die pharmazeutischen Präparate werden in an sich bekannten Lösungs-, Misch-, Granulier- oder Dragierverfahren hergestellt.
Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z. B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche oder tierische Öle in Betracht, wie Sonnenblumenöl und Lebertran. Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspension oder Emulsion gebracht. Als Lösungsmittel kommen z.B. in Frage: Wasser, physiologische Kochsalzlösung oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.
Ebenfalls möglich ist der Einsatz von injizierbaren Retardzubereitungen. Als Arzneiformen können z.B. ölige Kristallsuspensionen, Mikrokapseln, Rods oder Implantate verwendet werden, wobei die letzteren aus gewebeverträglichen Polymeren, insbesondere bioabbaubaren Polymeren, wie z.B. auf der Basis von Polymilchsäure-Polyglycolsäure-Copolymeren oder Humanalbumin sein.

Ein weiterer Gegenstand der vorliegenden Erfindung ist ein Verfahren zur Behandlung solcher Krankheiten, bei denen sich durch die Inhibierung von Aspartylproteasen eine Verbesserung erzielen läßt, besonders virale Infektionen, ganz besonders Infektionen mit Retroviren, die auf eine solche Behandlung ansprechen. Es beinhaltet die Verabreichung einer Verbindung der Formel I an einen infizierten Patienten. Das Verfahren betrifft insbesondere Infektionen durch HIV, Typ 1. Die Verabreichung einer effektiven Dosis kann oral, parenteral, trans-dermal, intravenös, intramuskulär, rectal, durch Einatmen erfolgen. Die Dosierungseinheit liegt zwischen 0,05 bis zu 100mg/kg Körpergewicht, typischerweise beträgt sie 50 bis 1000mg und wird ein bis zehn mal täglich bei akuter oder chronischer Infektion verabreicht. Die nötige Dosierung wird vom Fachmann leicht bestimmt und hängt von Alter, Gewicht und Zustand des Patienten und von der Applikationsart ab. Auch die Kombinationstherapie, wie in Eur. Pat. Appl. No. 337 714 S. 42-47 beschrieben ist möglich.

Durch die nachfolgenden Ausführungsbeispiele sowie durch den Inhalt der Patentansprüche soll die vorliegende Erfindung näher erläutert werden.

### Beispiele

### 1) 2,6-Dibenzyl-1-Hydroxy-3,5-diaza-4-oxo-phosphorinon-1-oxid (Racemat aus [2S,6S] und [2R,6R].

Zu einer Lösung von 100 mg (0,293 mMol) Bis(1-amino-2-phenylethyl)phosphinsäure Hydrochlorid (Racemat) und 94,8 mg (0,733 mMol) Ethyldiisopropylamin in 100 ml absol. Dichlormethan (DCM) wurden langsam 104,5 mg (0,64 mMol) N,N'-Carbonyldiimidazol (CDI) in 3 ml absol. DCM zugetropft. Es wurde 48 Stunden bei Raumtemperatur (RT) gerührt, dann nochmals 104,5 mg (0,64 mMol) CDI zugegeben. Nach weiteren 24 Stunden wurde das Lösungsmittel im Vakuum abgedampft, der Rückstand zwischen DCM und Wasser verteilt, die wässrige Phase mit 1N HCl auf pH 3 eingestellt, die Phasen wechselseitig mit DCM und Wasser gewaschen, die organische Phase getrocknet, filtriert und eingeengt. Der Rückstand wurde zwischen Ethylacetat (EE) und Wasser verrührt und abgesaugt.
Ausbeute: 12 mg,
MS (ES⁺): 331 (M+H⁺, 30 %)

### 2) 2,6-Dibenzyl-1-Methoxy-3,5-diaza-4-oxo-phosphorinon-1-oxid (Racemat aus [2S,6S] und [2R,6R].

Zu 169,8 mg (0,533 mMol) Bis(1-amino-2-phenylethyl)phosphinsäuremethylester (Racemat) in 350 ml absol. DCM wurden langsam 129,7 (0,8 mMol) CDI in 4 ml absol. DCM zugetropft. Es wurde 24 Stunden bei RT gerührt, dann weitere 86,4 mg CDI in 2 ml DCM zugegeben und nochmals 48 Stunden bei RT gerührt. Anschließend wurde wie in Beispiel 1 beschrieben aufgearbeitet (pH 5 bis 6), der Rückstand durch Chromatographie an Kieselgel DCM/Methanol 96:4).
Ausbeute: 61,2mg;
MS (El): 345 (M+H⁺, 100);
¹H-NMR (200 MHz, DMSO): 2,70-3,09 (m, 4H, CH₂-Ar); 3,46 & 3,51 (je s, 3H, OCH₃); 3,48-3,62 (m, 1H, P-CH); 3,66-3,82 (m, 1H, P-CH); 5,96 (dt, 1H, NH); 6,40 (dt, 1H, NH); 7,18-7,37 (m, 10H, Ar-H).

### 3) 1-Hydroxy-3,5-diaza-4-oxo-phosphorinon-1-oxid-Triethylammoniumsalz

Zu einer Lösung von 340 mg (1 mMol) Bis(N-trimethylsilylaminomethyl)-O-trimethylsilylphospinat (L. Maier, J. Organomet. Chem. 178 (1979) 157) in 50 ml absol. Toluol wurden bei 0°C zuerst 303 mg (3 mMol) Triethylamin, dann 622 ml Phosgen (20 % in Toluol) zugetropft, dann 3 Stunden bei 0°C und 20 Stunden bei RT gerührt. Das Reaktionsgemisch wurde mit 10 ml Wasser versetzt und unter Vakuum zur Trockene eingeengt. Der Rückstand wurde mit Methanol verrührt, filtriert und an Kieselgel chromatographiert (DCM/Methanol 9:1, dann 1:1). Das Produkt wurde mit Ether verrieben und abgesaugt.
Ausbeute: 35,2 g
MS (ES⁺): m/e = 151 (M+H⁺, 10 %), 102 (100 %)
¹H-NMR (200 MHz, DMSO): 5,98 (d, 2H, NH); 2,90 (dd, J₁ = 25Hz, J₂ = 4Hz, 4H, P-CH₂)

### 6) [2R,6S]-Dibenzyl-1-Hydroxy-3,5-diaza-4-oxo-phosphorinon-1-oxid

Synthese analog Beispiel 1 aus (0,293 mMol) Meso-Bis(1-amino-2-phenylethyl)phosphinsäure Hydrochlorid.
Ausbeute: 46 %;
MS (FAB): m/e = 353 (M+Na⁺), 331 (M+H⁺);
¹H-NMR (200MHz, DMSO): 2,64-2,90 (m, 2H, CH₂-Ar); 2,98-3,19 (m, 2H, CH₂-Ar); 3,44-3,63 (m, 2H, P-CH₂); 5,90 (d, 2H, NH); 7,13-7,40 Im, 10H, Ar-H).

### 7) [2R,6S]-Dibenzyl-1-methoxy-3,5-diaza-4-oxo-phosphorinon-1-oxid

Synthese analog Beispiel 2 aus Meso-Bis(1-amino-2-phenylethyl)phosphinsäuremethylester;
Ausbeute: 49 %;
MS (FAB/LiCl): m/e = 351 (M+Li⁺, 80 %);
¹H-NMR (200 MHz, DMSO): 2,74-3,10 (m, 4H, CH₂-Ar); 3,15 & 3,64 (je d, 3H, OCH₃); 3,59-3,97 (m, 2H, P-CH); 5,96 (dt, 1H, NH); 6,07 & 6,31 (je d, 2H, NH); 7,16-7,40 (m, 10H, Ar-H).

### 8) ([2R],3,5,[6S,])-Tetrabenzyl-1-benzyloxy-3,5-diaza-4-oxo-phosphorinon-1-oxid (nicht erfindungsgemäß)

15,4 mg (0,32 mMol) NaH 50 % wurden in 3 ml absol. Dimethylsulfoxid (DMSO) suspendiert und erst 1,5 Stunden bei 65°C gerührt, dann wurde auf 10°C abgekühlt. Es wurden 10,5 mg (0,032 mMol) [2R,6S]-Dibenzyl-1-Hydroxy-3,5-diaza-4-oxo-phosphorinon-1-oxid (Beispiel 6) in 1,5 ml absol. DMSO zugegeben, dann 10 Minuten bei 15°C gerührt und anschließend 109,5 mg (0,64 mMol) Benzylbromid zugegeben. Es wurde 17 Stunden bei RT gerührt, dann unter Kühlen 38,4 mg (0,64 mMol) Eisessig/Toluol auf einmal zugegeben. Das Lösungsmittel wurde im Vakuum abdestilliert, der Rückstand zwischen EE und Wasser verteilt. Die Phasen wurden getrennt und wechselseitig mit EE und Wasser, dann mit wässriger NaCI-Lösung gewaschen. Die organische Phase wurde getrocknet, filtriert und eingeengt. Der Rückstand an Kieselgel chromatographiert. (Toluol/EE 7:3).
Ausbeute: 7,2 mg;
MS (ES⁺): m/e = 601 (M+H⁺, 100 %);

### 9) ([2R],3,5,[6S,])-Tetrabenzyl-1-methoxy-3,5-diaza-4-oxo-phosphorinon-1-oxid

Synthese analog Beispiel 8 aus [2R,6S]-Dibenzyl-1-methoxy-3,5-diaza-4-oxo-phosphorinon-1-oxid (Beispiel 7). Chromatographie an Kieselgel mit Toluol/EE 95:5, bis 6:4.
Ausbeute: 76 %;
MS (FAB): m/e = 525 (M+H⁺, 100 %);
¹H-NMR (200 MHz, DMSO): 2,92 (d, 3H, OCH₃); 2,98-3,15 (m,2H, CH₂-Ar); 3,36-3,72 (m, 6H, P-CH & N-CH₂ & CH₂-Ar); 5,17 (dd. 2H, N-CH₂); 6,95-7,43 (m, 20H, Ar-H).

### 10) ([2R],3,5,[6S,])-Tetrabenzyl-1-hydroxy-3,5-diaza-4-oxo-phosphorinon-1-oxid

### a) Verseifung mit Bromtrimethylsilan

32,4 mg ([2R],3,5,[6S,])-Tetrabenzyl-1-methoxy-3,5-diaza-4-oxo-phosphorinon-1-oxid (Beispiel 9) wurden in 3 ml Dioxan gelöst und 1,5 ml Bromtrimethylsilan zugegeben. Es wurde 20 Stunden bei RT gerührt, dann langsam Bromtrimethylsilan im Vakuum abdestilliert. Es wurde dreimal mit Toluol koevaporiert. Der Rückstand wurde 1 Stunde in 1ml THF/1ml Eisessig/1ml Wasser gerührt, dann mit Ethanol abgeraucht. Der Rückstand wurde an Kieselgel chromatographiert
(EE/Methanol 8:2).
Ausbeute: >95 %
MS (FAB): m/e = 533 (M+Na⁺, 30 %); 511 (M+H⁺, 70 %)
¹H-NMR (200 MHz, DMSO): 2,82-3,00 (m, 2H, CH₂-Ar); 3,08-3,40 (m, 2H, CH₂-Ar); 3,45-3,73 (m, 4H, P-CH & N-CH₂); 4,89 (d, 2H, N-CH₂); 7,03-7,36 (m, 20H, Ar-H).

### b) Verseifung mit HBr

1 mg ([2R],3,5,[6S,])-Tetrabenzyl-1-methoxy-3,5-diaza-4-oxo-phosphorinon-1-oxid (Beispiel 9) wurden in 1 ml HBr/Eisessig (33 %) gelöst und 6 Stunden bei 60°C gerührt. Das Lösungsmittel wurde im Vakuum abdestilliert, dann dreimal mit Toluol koevaporiert.
MS (FAB): m/e = 533 (M+Na⁺, 30 %); 511 (M+H⁺, 70 %)

### 11) ([2R],[6S,])-Dibenzyl-3,5-(4-Trimethylsilylethoxybenzyl)-1-methoxy-3,5-diaza-4-oxo-phosphorinon-1-oxid

Synthese analog Beispiel 9 aus [2R,6S]-Dibenzyl-1-methoxy-3,5-diaza-4-oxo-phosphorinon-1-oxid (Beispiel 7) und 4-Trimethylsilylethoxybenzylbromid;
Ausbeute: 64 %;
MS (FAB/LiCl): m/e = 851.5 (M+Li⁺, 100 %);

### 12) ([2R],[6S,])-Dibenzyl-3,5-(4-hydroxymethylbenzyl)-1-hydroxy-3,5-diaza-4-oxo-phosphorinon-1-oxid

40 mg (0,0473 mMol) ([2R],[6S,])-Dibenzyl-3,5-(4-Trimethylsilylethoxymethylbenzyl)- 1-methoxy-3,5-diaza-4-oxo-phosphorinon-1-oxid (Beispiel 11) wurden in 3 ml Dioxan gelöst. Dazu wurden 1,5 ml Bromtrimethylsilan gegeben und es wurde 20 Stunden bei Raumtemp, gerührt. Das Lösungsmittel wurde im Vakuum abdestilliert und es wurde mit 1ml Tetrahydrofuran (THF), 1ml Eisessig, 1 ml Wasser 1 Stunde verrührt, eingeengt, dann dreimal mit Toluol koevaporiert und der Rückstand an Kieselgel chromatographiert (EE/Methanol 8:2 bis 6:4).
Ausbeute: 60 %;
MS (FAB): m/e = 593 (M+Na⁺, 50 %); 571 (M+H⁺, 20 %)

### 13) ([2R],[6S,])-Dibenzyl-3,5-(4-brommethylbenzyl)-1-hydroxy-3,5-diaza-4-oxo-phosphorinon-1-oxid

4,5 mg ([2R],[6S,])-Dibenzyl-3,5-(4-Trimethylsilylethoxymethylbenzyl)-1-methoxy-3,5-diaza-4-oxo-phosphorinon-1-oxid (Beispiel 11) wurden in 1 ml 33 % Hbr/Eisessig gelöst und 16 Stunden bei RT gerührt. Danach wurde dreimal mit Toluol koevaporiert. Der Rückstand wurde mit Ether verrührt und der Ether dekantiert.
MS (FAB): 699, 697, 695 (M+H⁺)

### 14) 2,6-Dibenzyl-3,5-(2-naphthylmethyl)-1-methoxy-3,5-diaza-4-oxo-phosphorinon-1-oxid

### (Racemat aus [2S,6S] und [2R,6R].

Synthese analog Beispiel 8 aus 2,6-Dibenzyl-1-Methoxy-3,5-diaza-4-oxo-phosphorinon-1-oxid (Racemat aus [2S,6S] und [2R,6R], Beispiel 2) und 2-Naphthylbrommethan.
MS (ES⁺): m/e = 625 (M+H⁺, 100 %);
¹H-NMR (200 MHz, DMSO): 2,78-3,10 (m, 4H, CH₂-Naphth); 3,20 (d, 3H, OCH₃); 3,35 & 3,64 (jew. d, 2H, P-CH); 3,84-4,12 (m, 2H, N-CH₂); 4,64-4,81 (m, 2H, N-CH₂); 7,02-8,00 (m, 28H, Ar-H).

### 15) 7-(RS)-Acetoxy-2(S), 6(S)-dibenzyl-1,4-dioxo-1-methoxy-1-phospha-3,5-diazepin

### 15a) (1-(N-Benzyloxycarbonyl-amino)-2(S)-phenethyl)-(1(RS)-acetoxy-2(S)-(N-benzyloxycarbonyl-amino)-phenpropyl)-(RS)-phosphinsäuremethylester

(1-Amino-2(S)-phenethyl)-(2(R)-amino-1(RS)-hydroxy-2-phenpropyl)-(RS)-phosphinsäuremethylester (2,9 g, 4,6 mMol) (Synthese analog B. Stowasser et. al. Tetrahedron Lett. 44 (1992) 6625) wird in einem Gemisch aus Pyridin und Essigsäureanhydrid (je 5 ml) 5 Stunden auf 50 bis 60°C erwärmt und anschließend eingeengt. Der Rückstand wird in Essigester aufgenommen und und mit gesättigter Ammoniumchloridlösung sowie gesättigter Natriumhydrogencarbonatlösung exthrahiert. Die Reinigung erfolgt durch Chromatographie an Kieselgel mit einem Toluol/Essigester-Gradienten (3:11:1).
Ausbeute: 2,9 g(4,4 mMol), 95 %.
MS (ES⁺): m/e = 672 (M+H⁺ = 673, 100 %)

### 15b) (1-Amino-2(S)-phenethyl)-(1(RS)-acetoxy-2(R)-amino-2-phenpropyl)-(RS)-phosphinsäuremethylester

(1-(N-Benzyloxycarbonyl-amino)-2(S)-phenethyl)-(1(RS)-acetoxy-2 (S)-(N-benzyloxycarbonyl-amino)-phenpropyl)-(RS)-phosphinsäuremethylester (2,9 g, 4,4 mMol) wird in wasserfreiem Ethanol (15 ml) gelöst, mit Palladium auf Kohle (5 %, 100 mg) versetzt und 5 Stunden in einer Wasserstoffatmosphäre gerührt. Nach 2 Stunden wird erneut Katalysator zugesetzt. Anschließend wird von dem Katalysator abfiltriert, eingeengt und an Kieselgel mit einem Essigester/Methanol-Gradienten (4:14:2) chromatographiert.
Ausbeute: 1,6 g, (3,9 mMol), 90 %.
FAB-MS (MeOH, NBA, LiCl): m/e = 404 (M+Li⁺ = 411, 100 %)

### 15c) 7-(RS)-Acetoxy-2(S), 6(S)-dibenzyl-1,4-dioxo-1-methoxy-1-phospha-3,5-diazepin

(1-Amino-2(S)-phenethyl)-(1(RS)-acetoxy-2(R)-amino-2-phenpropyl)-(RS)-phosphinsäuremethylester (1,6 g, 3,9 mMol) und Carbonyldiimidazol (720 mg, 4,2 mMol) werden in wasserfreiem Dichlormethan (50 ml) 45 Stunden bei Raumtemperatur gerührt und eingeengt. Zur Reinigung wird an Kieselgel mit einem Essigester/Methanol-Gradienten (6:14:1) chromatographiert.
Ausbeute: 1,14g (2,7 mMol), 70 %.
MS (ES⁺): m/e = 430 (M+H⁺ = 431, 100 %)

### 16) 7-(RS)-Acetoxy-2(S), 3,5,6(S)-tetrabenzyl-1,4-dioxo-1-methoxy-1-phospha-3,5-diazepin

Dimethylsulfoxid (50 ml) werden mit Natriumhydrid (500 mg) versetzt und 1,5 Stunden bei 65°C gerührt. Nach Zugabe von 7-(RS)-Acetoxy-2(S), 6(S)-dibenzyl-1,4-dioxo-1-methoxy-1-phospha-3,5-diazepin (1,14g, 2,7 mMol) wartet man 10 Minuten tropft dann Benzylbromid (3 ml) zu. Nach einer Stunde bei 30°C versetzt man mit Essigsäure, verdünnt mit Essigester und exthrahiert mit gesättigter Natriumhydrogencarbonatlösung. Zur Reinigung wird an Kieselgel mit einem Essigester/Methanol-Gradienten (25:14:1) chromatographiert.
Ausbeute: 1,0 g (1,6 mMol), 60 %.
FAB-MS (MeOH, NBA, LiCl): mle = 610 (M+Li⁺ = 617, 100 %)

### 17) 7-(RS)-Acetoxy-2(S), 3,5,6(S)-tetrabenzyl-1,4-dioxo-1-hydroxy-1-phospha-3,5-diazepin

7-(RS)-Acetoxy-2(S), 3, 5, 6(S)-tetrabenzyl-1,4-dioxo-1-methoxy-1-phospha-3,5-diazepin (1,0 g, 1,6 mMol) werden in einem Gemisch aus Dioxan (8 ml) und Bromtrimethylsilan (4 ml) 1 bis 2 Tage gerührt. Nach Einengen der Lösung wird zur Reinigung an Kieselgel mit einem Essigester/Methanol-Gradienten (4:1) mit 4 % Essigsäure chromatographiert.
Ausbeute: 530 mg (1,1 mMol), 65 %
FAB-MS (MeOH, NBA, LiCl): m/e = 582 (M+2Li⁺-H = 595, 100 %).

### 18) 2(S),3,5,6(S)-tetrabenzyl-1,4-dioxo-1-7-(RS)-dihydroxy-1-phospha-3,5-diazepin

7-(RS)-Acetoxy-2(S), 3,5,6(S)-tetrabenzyl-1,4-dioxo-1-hydroxy-1-phospha-3,5-diazepin (530 mg, 1,1 mMol) werden in wasserfreiem Dichlormethan (3 ml) gelöst, auf 78°C abgekühlt und mit einer Lösung von Diisobutylaluminiumhydrid in Dichlormethan (2,5 Equivalente) versetzt. Nach 2 Stunden wird Methanol (1 ml) zugesetzt, mit Essigester verdünnt, mit einer gesättigten Kaliumnatriumtartratlösung exthrahiert und zur Reinigung an Kieselgel mit einem Essigester/Methanol-Gradienten (5:12:1) mit 4 % Essigsäure chromatographiert.
Ausbeute: 430 mg (0,8 mMol), 72 %.
FAB-MS (MeOH, NBA, LiCl): m/e = 540 (M+2Li⁺-H = 553, 100 %).

### 19) 2,6-Dibenzyl-3,5-(2-naphthylmethyl)-1-hydroxy-3,5-diaza-4-oxo-phosphorinan-1-oxid (Racemat aus [2S,6S] und [2R,6R].

Synthese analog Beispiel 10a aus 2,6-Dibenzyl-3,5-(2-naphthylmethyl)-1-methoxy-3,5-diaza-4-oxo-phosphorinan-1-oxid (Racemat aus [2S,6S] und [2R,6R], Beispiel 14);
Ausbeute: 90 %
MS (ES⁺): m/e = 633 (M+Na⁺, 10%), 611 (M+H⁺, 30%);
¹H-NMR (200 MHz, CDCl₃): 2,63-3,68 (m, 6H, Ar-CH₂ & P-CH); 3,40-3,69 (m, 2H, N-CH₂); 4,60-4,85 (m, 2H, N-CH₂); 6,62-7,88 (m,28 H, Ar-H).

### 20) 2,5-Dibenzyl-1-hydroxy-3,5-diaza-4-oxo-phosphorinan-1-oxid (Racemat aus [2S,6S] und (2R,6R].

Synthese analog Beispiel 10a aus 2,6-Dibenzyl-1-methoxy-3,5-diaza-4-oxo-phosphorinan-1-oxid (Racemat aus [2S,6S] und (2R,6R] (Beispiel 2) durch Umsetzung mit Bromtrimethylsilan.
MS (ES⁺): m/e = 331 (M+H⁺, 30%);
¹H-NMR (200 MHz, DMSO): 2,48-2,75 (m, 2H, P-CH); 2,97-3,21 (m, 4H, Ar-CH₂); 4,87 (d, 2H, NH); 7,10-7,39 (m, 10H, Ar-H).

### 22) ([2R,[6S])-Dibenzyl-3,5-(4-brommethylbenzyl)-1-hydroxy-3,5-diaza-4-oxo-phosphorinan-1-oxid

### (Racemat aus [2S,6S] und [2R,6R]

Synthese analog Beispiel 10b, aus ([2R],[6S])-Dibenzyl-3,5-(4-(4-methoxyphenoxymethylbenzyl)-1-hydroxy-3,5-diaza-4-oxo-phosphorinan-1-oxid (Racemat aus [2S,6S] und [2R,6R], abweichend jedoch wurde 74 h bei 0°C gerührt.
Ausbeute: 88 %
MS (FAB): m/e = MS (FAB): 699,1, 697,1, 695,1 (M+H⁺)

### 23) ([2R,[6S])-Dibenzyl-3,5-(4-hydroxymethylbenzyl)-1-hydroxy-3,5-diaza-4-oxo-phosphorinan-1-oxid

### (Racemat aus [2S,6S] und [2R,6R]

6 mg ([2R,[6S])-Dibenzyl-3,5-(4-brommethylbenzyl)-1-hydroxy-3,5-diaza-4-oxo-phosphorinan-1-oxid (Beispiel 22) wurde in 1 ml Dioxan/Wasser gelöst und mit 13 mg CaCO₃ versetzt. Anschließend wurde 3 h bei 110°C gerührt. Das Lösungsmittel wurde verdampft, der Rückstand mit 10 ml Essigester versetzt, es wurden 0,39 ml 1 N HCI zugegeben und 10 min gerührt. Die Phasen wurden getrennt und wechselseitig mit Essigester und NaCI-Lösung gewaschen. Die organische Phase wurde getrocknet, filtriert und eingeengt. Der Rückstand wurde mit n-Pentan verrieben und abgesaugt.
Ausbeute: 4 mg; MS (FAB): 593,2 (M+Na⁺); 571,2 (M+H⁺).

### 24) Test auf Inhibition der HIV-Protease

Der Test auf Inhibition der HIV-Protease wurde wie in EP 0 428 849 A2 beschrieben durchgeführt. Die nachfolgenden IC₅₀-Werte geben an, bei welcher Konzentration die Aktivität des Enzyms um die Hälfte vermindert ist;
Beispiel 19: IC₅₀ = 320 nM
Beispiel 12: IC₅₀ = 4800 nM
Beispiel 13: IC₅₀ = 18000 nM
Beispiel 10: IC₅₀ = 50000 nM
Beispiel 23: IC₅₀ = 240 nM

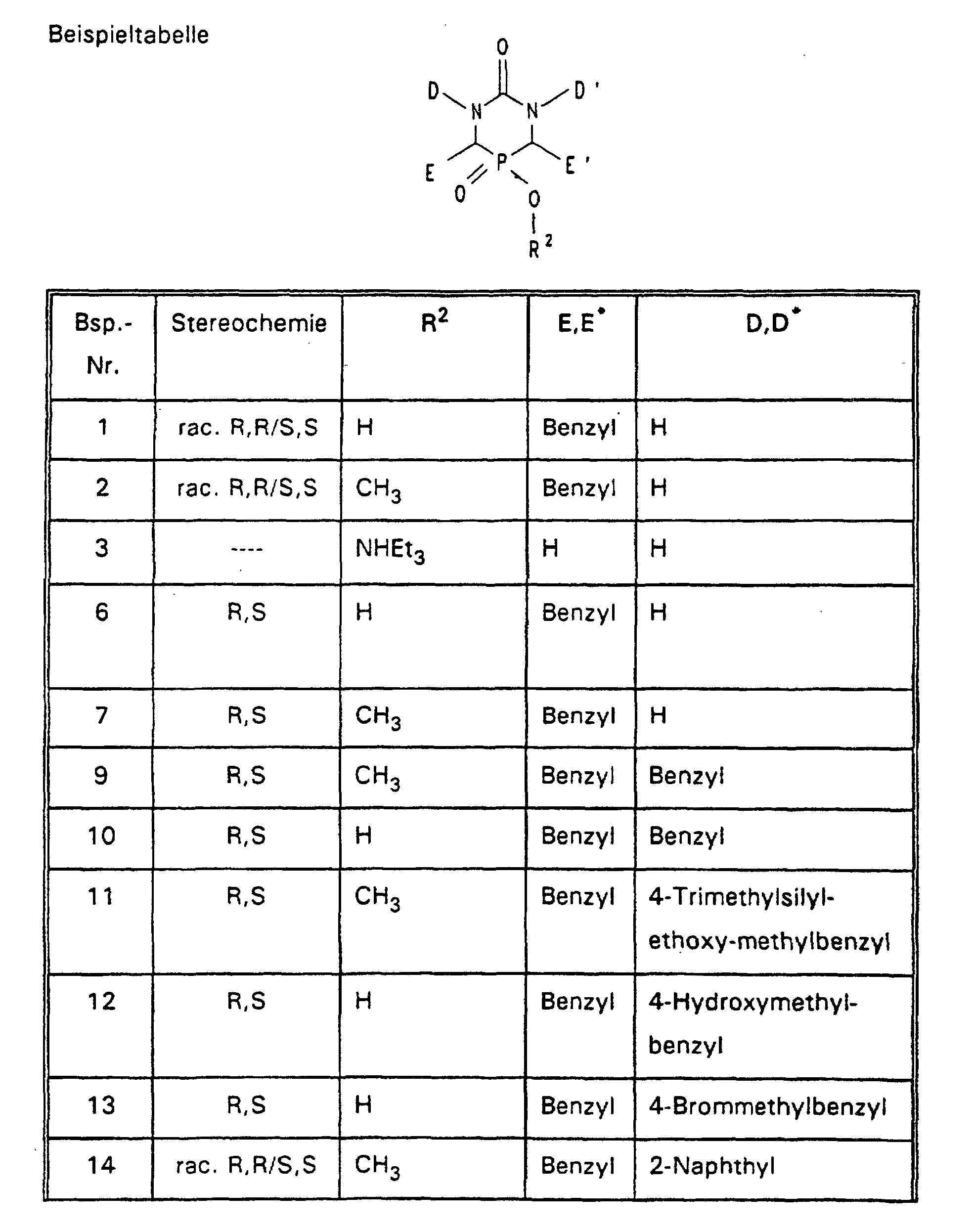

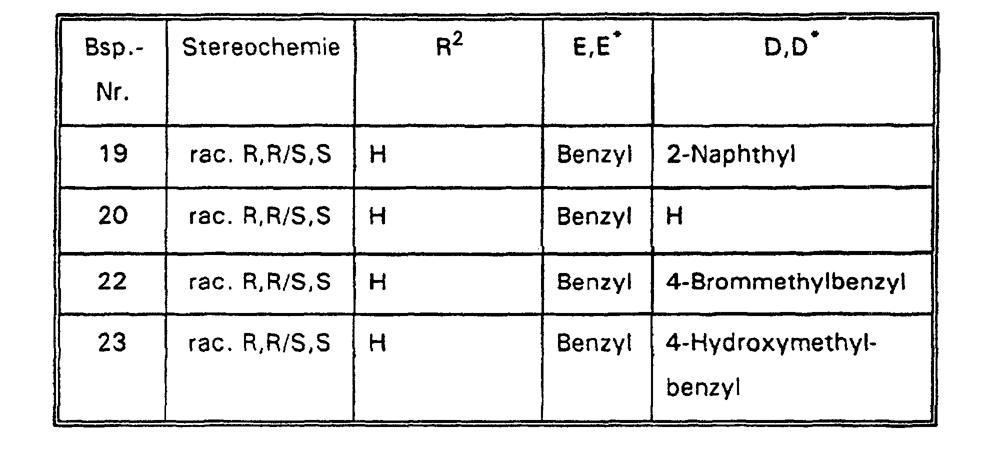

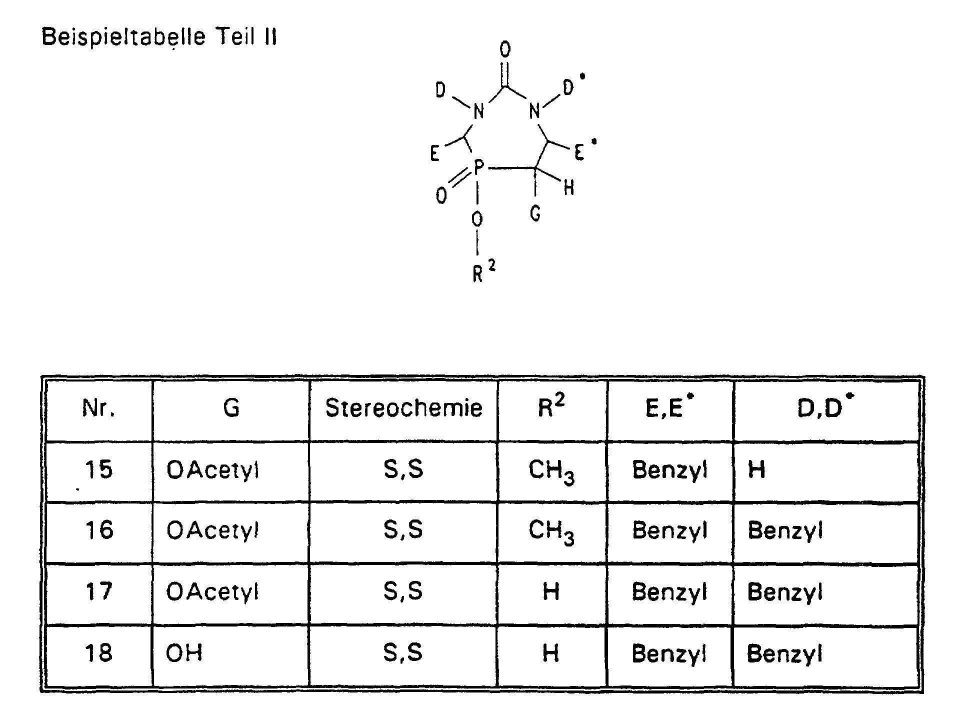

## Patentansprüche

1. Verbindungen der Formel I 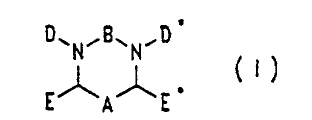 worin A für einen Rest der Formel IIa, IIb oder IIc 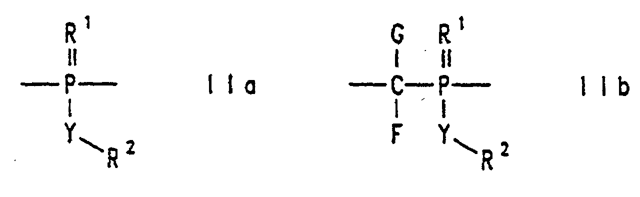 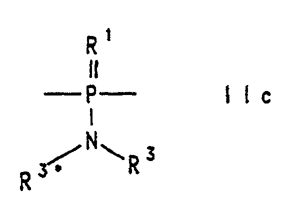 steht;
Y für Sauerstoff oder Schwefel steht;
R¹ für Sauerstoff oder Schwefel steht,,
R2 für
- Wasserstoff,
- (C₁-C₄)-Alkyl,
- ein Äquivalent eines physiologisch verträglichen Salzes, oder
- ein Rest einer Phosphinsäure-Prodrug,
steht;
R³ und R^{3*} Wasserstoff bedeuten;
G und F stehen unabhängig voneinander für
- Wasserstoff
- Fluor,
- Hydroxy,
- O-R⁴,
mit der Maßgabe, daß wenn G für Hydroxy steht, F Wasserstoff bedeuten soll;
R⁴ steht für Wasserstoff oder (C₁-C₄)-Acyl;
B steht für einen Rest der Formel IIIa, L bedeutet Sauerstoff; 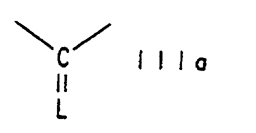
D und D* unabhängig voneinander für R⁶ oder R⁶-CH₂- stehen, wobei R⁶ für (C₆-C₁₂)-Aryl, unsubstituiert oder einfach substituiert mit R⁷
wobei R⁷ unabhängig voneinander für
- R⁸
- OR⁸,
- F, -Cl, -Br steht, wobei
R⁸ für Wasserstoff,
(C₁-C₆)-Alkyl, unsubstituiert oder mit Hydroxy oder Br substituiert und
E und E * Wasserstoff oder
Benzyl bedeuten.

2. Verbindungen der Formel I gemäß Anspruch 1 in denen
R² für
- Wasserstoff;
- (C₁-C₄)-Alkyl oder
- ein Äquivalent eines physiologisch verträglichen Salzes steht, und D steht für Phenyl oder Benzyl, substituiert mit R⁸ wobei
R⁸ Wasserstoff,
(C₁-C₃)-Alkyl, gegebenenfalls mit Hydroxy oder Br substituiert, bedeutet.

3. Verfahren zur Herstellung von Verbindungen der Formel I gemäß einem oder mehreren der Ansprüche 1-2, **dadurch gekennzeichnet, daß**
eine Verbindung der Formel IV 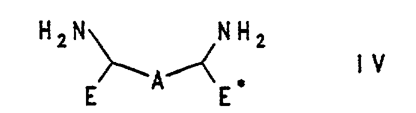 worin A, E und E* die obengenannten Bedeutungen haben, mit aktivierten Kohlensäure- oder Oxalsäurederivaten zu zyklischen Harnstoffen bzw. zyklischen Oxalsäurediamiden der Formel V 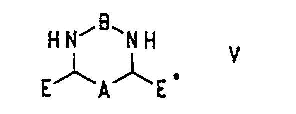 worin A, E, E* und B die oben genannte Bedeutung haben, umgesetzt wird und anschließend die erhaltenen Verbindungen zu Verbindungen der Formel I alkyliert werden oder daß die Verbindungen der Formel IV alkyliert werden und anschließend die erhaltenen Verbindungen zu Verbindungen der Formel I zyklisiert werden.

4. Verbindungen der Formel I gemäß einem oder mehreren der Ansprüche 1 - 2 als Arzneimittel.

5. Arzneimittel, enthaltend eine wirksame Menge mindestens einer Verbindung der Formel I gemäß einem oder mehreren der Ansprüche 1 - 2.

6. Verfahren zur Herstellung eines Arzneimittels gemäß Anspruch 5, **dadurch gekennzeichnet, daß** die wirksame Menge einer Verbindung der Formel 1 mit üblichen pharmazeutischen Hilfsstoffen in eine geeignete Darreichungsform gebracht wird.

7. Verwendung von Verbindungen der Formel I gemäß einem oder mehreren der Ansprüche 1-2 zur Herstellung von Arzneimitteln zur Behandlung von Krankheiten, die durch Viren verursacht werden, insbesondere von Krankheiten, die durch das HIV verursacht werden.

## Claims

1. A compound of the formula I 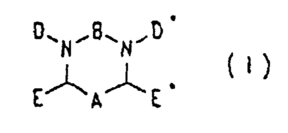 in which A is a radical of the formula IIa, IIb or IIc 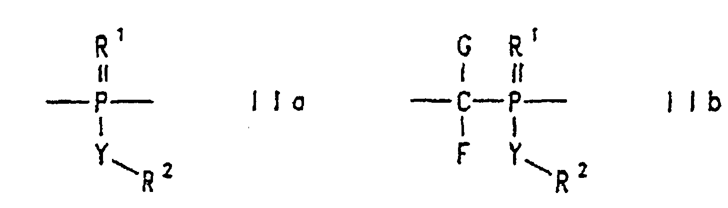 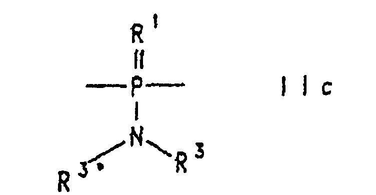
Y is oxygen or sulfur;
R¹ is oxygen or sulfur;
R² is
- hydrogen,
- (C₁-C₄)-alkyl,
- an equivalent of a physiologically tolerated salt, or
- a residue of a phosphinic acid prodrug;
R³ and R³* are hydrogen;
G and F are, independently of each other,
- hydrogen,
- fluorine,
- hydroxyl,
- O-R⁴,
with the proviso that if G is hydroxyl, F should be hydrogen;
R⁴ is hydrogen or (C₁-C₄)-acyl;
B is a radical of the formula IIIa, L is oxygen; 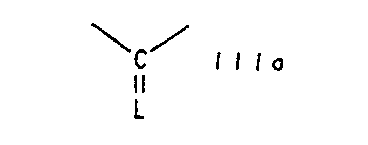
D and D* are, independently of each other, R⁶ or R⁶-CH₂-, where R⁶ is (C₆-C₁₂)-aryl which is unsubstituted or substituted once by R⁷,
where R⁷ is, independently of each other,
- R⁸,
- OR⁸,
- F, -Cl or -Br, where
R⁸ is
hydrogen,
(C₁-C₆)-alkyl which is unsubstituted or substituted by hydroxyl or Br, and
E and E* are hydrogen, or benzyl.

2. A compound of the formula I as claimed in claim 1, in which
R² is
- hydrogen;
- (C₁-C₄)-alkyl, or
- an equivalent of a physiologically tolerated salt,
and D is phenyl or benzyl which are substituted by R⁸, where
R⁸ is hydrogen,
(C₁-C₃)-alkyl, optionally substituted by hydroxyl or Br.

3. A process for-preparing compounds of the formula I as claimed in one or both of claims 1 - 2, which comprises a compound of the formula IV 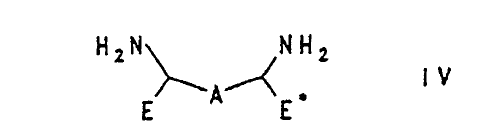 in which A, E and E* have the abovementioned meanings, being reacted with activated carbonic acid derivatives or oxalic acid derivatives to form cyclic ureas or cyclic oxalic acid diamides of the formula V 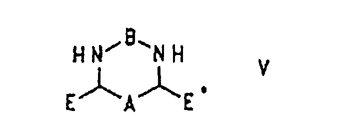 in which A, E, E* and B have the abovementioned meaning, and the resulting compounds subsequently being alkylated to form compounds of the formula I, or which comprises the compounds of the formula IV being alkylated and subsequently the resulting compounds being cyclized to form compounds of the formula I.

4. A compound of the formula I as claimed in one or both of claims 1 - 2 as pharmaceuticals.

5. A pharmaceutical, containing an effective quantity of at least one compound of the formula I as claimed in one or both of claims 1 - 2.

6. A process for preparing a pharmaceutical as claimed in claim 5, which comprises bringing the effective quantity of a compound of the formula I into a suitable form for administration together with customary pharmaceutical auxiliary substances.

7. Use of compounds of the formula I as claimed in one or both of claims 1 - 2 for preparing pharmaceuticals for treating diseases which are caused by viruses, in particular diseases which are caused by HIV.

## Revendications

1. Composés de formule I 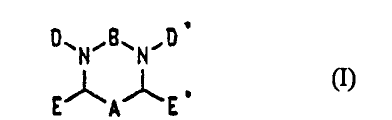 dans laquelle A représente un reste de formule IIa, IIb ou IIc 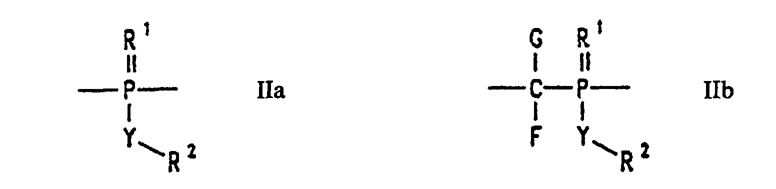 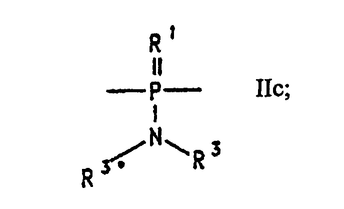
Y représente un atome d'oxygène ou de soufre;
R¹ représente un atome d'oxygène ou de soufre;
R² représente un atome d'hydrogène, un reste alkyle en C₁-C₄, un équivalent d'un sel physiologiquement acceptable, ou un reste d'un précurseur de médicament de type acide phosphinique,
R³ et R³* représentent un atome d'hydrogène;
G et F représentent, indépendamment l'un de l'autre, un atome d'hydrogène, un atome de fluor, un reste hydroxy, un reste -O-R⁴, à condition que, lorsque G est un reste hydroxy, F soit un atome d'hydrogène;
R⁴ représente un atome d'hydrogène ou un reste acyle en C₁-C₄;
B représente un reste de formule IIIa, dans lequel L représente un atome d'oxygène: 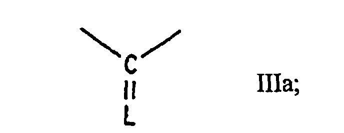
D et D* représentent, indépendamment l'un de l'autre, un reste R⁶ ou R⁶-CH₂-, où R⁶ est un reste aryle en C₆-C₁₂ non substitué ou substitué une fois par R⁷, les R⁷ étant indépendamment un reste R⁸, OR⁸, F, Cl ou Br, où
R⁸ représente un atome d'hydrogène ou un reste alkyle en C₁-C₆ non substitué ou substitué par hydroxy ou Br, et
E et E* représentent un atome d'hydrogène ou un reste benzyle.

2. Composés de formule I selon la revendication 1, dans lesquels
R² représente un atome d'hydrogène, un reste alkyle en C₁-C₄ ou un équivalent d'un sel physiologiquement acceptable, et
D représente un reste phényle ou benzyle substitué par R⁸, R⁸ étant un atome d'hydrogène ou un reste alkyle en C₁-C₃ éventuellement substitué par hydroxy ou Br.

3. Procédé de préparation de composés de formule I selon l'une ou plusieurs des revendications 1 - 2, **caractérisé en ce que** l'on fait réagir un composé de formule IV 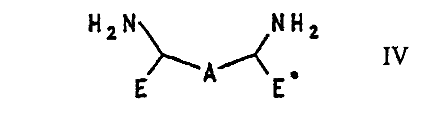 dans laquelle A, E et E* ont la signification indiquée ci-dessus, avec des dérivés d'acide carbonique ou d'acide oxalique activés pour obtenir des urées cycliques ou des diamides d'acide oxalique cycliques de formule V 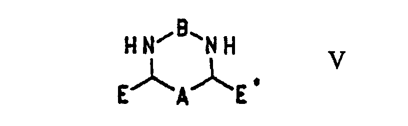 dans laquelle A, E, E* et B ont la signification donnée ci-dessus, puis on alkyle les composés obtenus pour obtenir des composés de formule I, ou **en ce que** l'on alkyle les composés de formule IV, puis on cyclise les composés obtenus pour obtenir des composés de formule I.

4. Composés de formule I selon l'une des revendications 1 - 2 comme médicaments.

5. Médicaments contenant une quantité active d'au moins un composé de formule I selon l'une ou plusieurs des revendications 1-2.

6. Procédé de préparation d'un médicament selon la revendication 5, **caractérisé en ce que** l'on met la quantité active d'un composé de formule I sous une forme d'administration appropriée avec des additifs pharmaceutiques classiques.

7. Utilisation de composés de formule I selon l'une ou plusieurs des revendications 1 - 2 pour la préparation de médicaments destinés au traitement de maladies dues à des virus, en particulier de maladies dues au VIH.
